# FASCICULE DE BREVET EUROPEEN

(11) **EP 1 320 552 B1**
(45) Date de publication et mention de la délivrance du brevet: **29.03.2006**
(21) Numéro de dépôt: 01972206.5
(22) Date de dépôt: 25.09.2001
(51) Int. Cl.: C07K 16/00, A61K 39/395, C07K 16/18

(54) **PROCEDE DE PRODUCTION D'IMMUNOGLOBULINES ANTI-THYMOCYTES HUMAINS**
HERSTELLUNGSVERFAHREN VON IMMUNGLOBULINEN, DIE GEGEN MENSCHLISCHE THYMOZYTEN GERICHTET SIND
METHOD FOR PRODUCING HUMAN ANTI-THYMOCYTE IMMUNOGLOBULINS

(30) Priorité: 28.09.2000 FR 0012384
(43) Date de publication de la demande: 25.06.2003
(73) Titulaire: IMTIX Sangstat, 69348 Lyon Cédex 07 (FR)
(72) Inventeur: TIOLLIER, Jérôme, F-69005 Lyon (FR); SORLIN, Laurent, F-69670 Vaugneray (FR)
(74) Mandataire: Bernasconi, Jean Raymond
(86) Numéro de dépôt international: PCT/FR2001/002972
(87) Numéro de publication internationale: WO 2002/026830

(56) Documents cités:
- US-A- 4 364 935
- BEAUFIGEAU M ET AL: "Seven-year experience with rabbit antithymocyte globulin after cardiac transplantation at the Montreal Heart Institute." TRANSPLANTATION PROCEEDINGS, vol. 29, no. 7A, novembre 1997 (1997-11), pages 10S-12S, XP001002862 Symposium on North American Experience with Rabbit Anti-Human Thymocyte Globulin;Mont Tremblant, Quebec, Canada; January 31, 1997 ISSN: 0041-1345
- SKOPINSKA-ROZEWSKA E ET AL: "SEARCH FOR THE BEST SCHEDULE FOR PRODUCTION OF NONTOXIC ANTI THYMOCYTE GLOBULIN" MATERIA MEDICA POLONA, vol. 13, no. 3, 1981, pages 187-194, XP001002872 ISSN: 0025-5246
- VERSCHOOR E J ET AL: "VACCINATION AGAINST FELINE IMMUNODEFICIENCY VIRUS USING FIXED INFECTED CELLS" VETERINARY IMMUNOLOGY AND IMMUNOPATHOLOGY, AMSTERDAM, NL, vol. 46, no. 1/2, 1 mai 1995 (1995-05-01), pages 139-149, XP000609769 ISSN: 0165-2427
- PREVILLE X ET AL: "A quantitative flow cytometry assay for the preclinical testing and pharmacological monitoring of rabbit antilymphocyte globulins (rATG)" JOURNAL OF IMMUNOLOGICAL METHODS, ELSEVIER SCIENCE PUBLISHERS B.V.,AMSTERDAM, NL, vol. 245, no. 1-2, 1 novembre 2000 (2000-11-01), pages 45-54, XP004218807 ISSN: 0022-1759

## Description

La présente invention se rapporte à un procédé de production d'imunoglobulines anti-thymocytes humains.

Les anticorps ou immunoglobulines (1g) anti-thymocytes humains sont connus comme immunosuppresseurs sélectifs. Ils agissent sur la réponse immunitaire en diminuant par déplétion, selon différents mécanismes, la quantité des lymphocytes circulants du sang et des différents tissus lymphoïdes et probablement en bloquant ou modulant leurs récepteurs. Leur utilisation est indiquée dans le cadre des transplantations d'organes, pour la prévention et le traitement des rejets de greffes, ainsi que pour le traitement de la réaction du greffon contre l'hôte aiguë. Ces immunoglobulines ont également été proposées dans le traitement de l'aplasie médullaire.

Des préparations de ce type d'immunoglobulines-étaient initialement obtenues par injection de cellules lymphatiques chez l'animal, lapin ou cheval par exemple, puis par extraction des immunoglobulines anti-thymocytes à partir du sérum immun. Toutefois, le niveau de pureté de ces préparations était insuffisant pour rendre ces immunoglobulines utilisables, notamment en thérapie (Guttman, R.D., 1967, J. Exp. Med., 126:1099-1127). Des immunoglobulines anti-érythrocytes humains ou anti-membrane basale étaient en particulier produites par l'animal au cours de l'immunisation. Plusieurs hypothèses expliquent l'apparition de ces immunoglobulines : soit les suspensions thymocytaires renferment des traces d'hématies (Rolland, J.M, 1972, Pathology, 4(2) : 85-122), soit il existe des antigènes de surface communs entre les cellules T, B et les érythrocytes, ce qui à l'heure actuelle est l'hypothèse la plus vraisemblable.

Des techniques ont alors été développées afin d'améliorer la pureté de la préparation. Ces techniques prévoyaient une étape d'hémadsorption, à savoir une adsorption sur des hématies humaines.

Cette étape d'hémadsorption permet l'élimination de la majeure partie des anticorps indésirables, tels que les anticorps anti-érythrocytes présents dans le sérum immun initial.

Une étape d'élimination des anticorps anti-tissulaires sur tissus humains (comme le placenta) était également parfois prévue, afin d'adsorber les anticorps anti-membrane basale en particulier.

Plusieurs immunoglobulines anti-thymocytes humains préparées selon ces techniques sont maintenant disponibles sur le marché, tels que la Thymoglobuline® (imtix-Sangstat), le Tecelac® (Biotest), ou encore l'ATGF® (Fresenius), ou l'ATGAM (Upjohn).

Néanmoins, les procédés décrits qui comprennent une étape d'adsorption sur hématies ou tissus humains présentent encore des inconvénients.

En particulier, on observe, lors de la mise en oeuvre de ces procédés, une diminution de 30% du taux de gammaglobulines contenues dans le sérum, et donc d'une partie non négligeable des anticorps d'intérêt. En outre, l'élimination des anticorps anti-érythrocytes indésirables n'est pas totale. Or, ceux-ci seraient susceptibles de provoquer l'apparition d'anémies hémolytiques chez les patients traités. Enfin, l'utilisation de matériel humain, tels que des érythrocytes, même purifiés et sécurisés, dans le procédé d'isolement des immunoglobulines anti-thymocytes humains augmente le risque potentiel d'infection.

Les auteurs de la présente invention ont donc cherché à mettre au point un procédé amélioré pour la production d'immunoglobulines anti-thymocytes humains.

La présente invention se rapporte à un procédé sans étape d'adsorption sur du matériel biologique humain, pour l'obtention d'une préparation d'immunoglobulines anti-thymocytes suffisamment pure, pour une utilisation en thérapie notamment.

Plus précisément, l'invention a pour objet un procédé de production d'immunoglobulines anti-thymocytes humains purifiés qui comprend les étapes consistant à :
(a) injecter une préparation cellulaire de thymocytes humains à un animal exempt d'organismes pathogènes spécifiés (EOPS) ;
(b) recueillir le sérum immun produit par l'animal ;
(c) isoler les immunoglobulines anti-thymocytes humaines du sérum, sans hémasorption sur hématies ni adsorption sur tissus humains, stroma ou extraits grossiers de ces tissus.

De manière plus particulière, on entend par immunoglobulines du sérum" la fraction totale des gammaglobulines du sérum après immunisation. Les tissus humains peuvent etre du placenta notamment.

Les animaux EOPS (ou SPF pour « Specific-pathogen free ») sont des animaux d'élevage dont l'environnement ainsi que l'alimentation sont strictement contrôlés selon des normes sanitaires. On pade également d'animaux « au statut sanitaire contrôlé » (SCC). -Les animaux EOPS sont élevés dans des habitacles fiermés stérilisés, l'environnement d'air étant filtré, par exemple sur filtre HEPA (filtration stérilisante), et l'eau et les aliments étant décontaminés avant Introduction, ce qui permet l'élimination de tout élément pathogène de leur environnement direct (Yanabe et al. Exp. Animal 48(2). 101-106 (1999)). Le terme "pathogène" désigna ici un agent infectieux capable de causer une maladie clinique et/ou d'altérer la réponse biologique de l'animal au regard de l'utilisation souhaitée. Le statut EOPS se réfère à une liste (évolutive) de germes et de méthode de contrôle (clinique, sérologique, de culture ou histologique) pour dètecter les microorganismes ciblés ou au contraire démontrer leur absence.

On utilise de manière préférentielle la cheval ou la chèvre, de préférence encore le lapin, pour la production de sérum immun. En effet, seules des IgG1 sont présentes dans le sérum de lapin, ce qui facilite la processus de purification des anticorps. L'lgG de lapin montre en outre une forte affinité pour le récepteur Fe des humains, permettant le développement d'anticorps cytotoxiques puissants dirigés contre les cellules T humaines.

La préparation cellulaire de thymus humains peut être obtenue soit à partir de cellules de lignées en culture, soit à partir de thymocytes frais qui sont purifiés préférentiellement à partir de fragments de thymus humains soit éventuellement à partir, par exemple, de suspensions de rate, d'amygdales, de ganglions lymphatiques, de trachée thoracique ou encore de sang périphérique. Les fragments de thymus humains peuvent être notamment facilement prélevés lors d'actes chirurgicaux, notamment à la suite de chirurgies cardiaques sur des enfants. Des tests virologiques sont réalisés sur le sang du donneur pour éviter toute contamination et éliminer tout fragment thymique contaminé, montrant un résultat sérologique positif.

L'injection des thymocytes humains et la collecte du sérum immun sur l'animal EOPS sont réalisées selon les techniques standards de l'homme du métier.

L'isolement des immunoglobulines anti-thymocytes humains du sérum permet l'élimination des protéines indésirables. Il peut être réalisé en mettant par exemple en oeuvre une chromatographie échangeuse d'ions, de préférence sur colonne et/ou une ou plusieurs précipitations.

De manière avantageuse, une telle précipitation peut être réalisée en deux étapes successives au moyen d'un réactif précipitant les immunoglobulines. Le réactif préférentiellement utilisé est le sulfate de sodium.

L'étape chromatographique permet la rétention des impuretés chargées par une résine échangeuse d'ions tels que des anions (DEAE). Les IgG quant à elles ne sont pas retenues par la colonne et sont évacuées rapidement de la colonne, ce qui permet de les récolter de manière sélective. Une chromatographie sur colonne d'affinité spécifique, éliminant les anticorps indésirables restant, est également avantageuse.

Sont également décrites les immunoglobulines anti-thymocytes humains isolées susceptibles d'être préparées par le procédé de l'invention.

Ces immunoglobulines peuvent également être désignées "immunoglobulines anti-lymphvcytes humains" puisqu'elles reconnaissent les lymphocytes humains lorsqu'elles sont mises en présence da ceux-ci, par exemple lors d'injections à des patients en vue d'une immunodéplétion.

La présente demande décrit également l'utilisation de ces immunoglobulines pour la fabrication d'un médicament destiné à diminuer la quantité de lymphocytes circulants du sang et des tissus lymphoïdes chez un patient humain.

Ces immunoglobulines sont particulièrement utiles dans le cadre des transplantations d'organes.

Les immunoglobulines anti-thymocytea humains préparées selon le procédé décrit plus haut présentent une plus grande activité spécifique que les immunoglobulines anti-thymocytes préparées au moyen d'un procédé incluant une étape d'hémadsorption, comme cela ressort dans l'exemple 2.1 présenté ci-après.

La suppression de l'étape d'adsorption sur matériel biologique humain permet la fabrication d'immunoglobulines en l'absence de tout contact avec un dérivé humain. Ceci conduit à l'élimination de toute contamination éventuelle virsle ou d'agents non conventionnels de type prion. En outre, on évite toute contamination associée à une hémadsorption, à savoir une contamination par l'hémoglobine relarguée par les hématies humaines lors de l'hémolyse engendrée par cette étape.

Le procédé de l'invention permet d'obtenir une préparation d'immunoglobulines anti-thymocytes dans laquelle le nombre d'immunoglobulines susceptibles de réactions croisées avec d'autres cellules sanguines (érythrocytes, neutrophiles, ...) est considérablement réduit De plus, la fait de ne pas utiliser d'hémadsorption dans la préparation des anticorps anti-thymocytes permet non seulement d'améliorer l'aspect sécuritaire mais réduit également la durée et le coût du procédé de préparation. En effet, de manière classique, l'hémadsorption utilise des globules rouges humains entiers, frais et formolés, et nécessite des quantités importantes de cellules à traiter, rendant l'obtention des immunoglobulines anti-thymocytes relativement contraignante au niveau industriel.

Les exemples suivants illustrent l'invention sans en limiter la portée.

### EXEMPLES :

### EXEMPLE 1 : Production d'immunoglobulines

### 1.1 Purification des thymocytes :

Des fragments de thymus humains sont, après prélèvement et dilacération, filtrés et mis en suspension.

La suspension cellulaire est ensuite filtrée sur un tissu en Nylon et soumise à une centrifugation à 1 800 tours/min à 5°C.

20 ml de Ficoll sont ajoutés à 10 ml de solution cellulaire contenant de 2 à 7.10⁹ cellules et l'ensemble est centrifugé à 2 000 tours/min à 5°C. Deux centrifugations subséquentes sont réalisées à 1 800 tours/min. Les préparations cellulaires sont ensuite stockées à 5°C pendant une nuit puis sont diluées avant injection à des lapins EOPS. Les thymocytes peuvent également être conservés par congélation.

### 1.2 Isolement des anticorps :

Le sérum immun de lapin est recueilli au cours de plusieurs saignées entre J20 et J30 et peut être congelé pour le stockage. Lors de la préparation, on forme des lots de sérum qui sont ramenés progressivement à température ambiante.

Ces lots de sérum de lapin sont décomplémentés afin d'éliminer les protéines du complément, en les portant à une température de 56°C ± 2°C pendant une durée de 30 à 45 min.

Le sérum de lapin est purifié pour la fraction d'immunoglobulines par une chromatographie sur une résine échangeuse d'anions (DEAE) à température ambiante suivie de deux précipitations au sulfate de sodium.

Après une nouvelle étape de filtration, de concentration et diafiltration, les immunoglobulines anti-thymocytes sont pasteurisées à une température de 60°C pendant 10 heures afin d'en assurer la sécurité virale.

Selon une variante, on peut effectuer les étapes de filtration, concentration, diafiltration et précipitation de la fraction immunoglobuline (par exemple fractionnement alcoolique de type COHN ou sulfate d'ammonium) avant l'étape de chromatographie.

La solution d'immunoglobulines peut, après formulation et stérilisation par filtration, être conservée à l'état liquide dans une solution de 5 à 25 mg/ml ou selon une variante à l'état lyophilisé. Une série de contrôles de qualité est exécutée incluant des tests physicochimiques, des tests de sécurité (présence d'agents pyrogènes, d'activité antiplaquettaire), des testés de stérilité et de pureté ainsi que des tests d'activités lymphocytotoxiques (inhibition de formation de rosettes in vitro et survie de greffon de peau allogénique chez le singe).

### EXEMPLE 2 :

### Analyse comparative de l'efficacité et de l'innocuité des immunoglobulines obtenues par le procédé de l'invention vis-à-vis des immunoglobulines classiques (obtenues par hémadsorption).

### 3.1 Mesure de l'activité spécifique in vitro

Deux lots d'immunoglobulines ont été utilisés pour l'étude comparative de l'activité spécifique : un lot d'immunoglobulines anti-thymocytes. (ATG) standard et un lot d'ATG non hémadsorbées.

Pour déterminer l'activité spécifique des ATG, c'est-à-dire pour évaluer la quantité d'immunoglobulines capables de se lier aux lymphocytes humains, on utilise une technique de cytométrie de flux couplée à une immunofluorescence indirecte. Du sang total (contenant l'ensemble des cellules sanguines) de singe est incubé avec les lots d'ATG à différentes concentrations. L'excès d'immunoglobulines non fixées est rincé et un second anticorps marqué à l'isothiocyanate de fluorescence (FITC) est ajouté pour venir se fixer sur les ATG liées. Ensuite, le taux de liaison des ATG à la surface des cellules est révélé après différenciation des cellules en fonction de leur taille grâce à la cytométrie de flux.

Les résultats montrent une plus grande activité spécifique des ATG non hémadsorbés. Ainsi, pour obtenir une même liaison aux lymphocytes, il faut 2,73 µg/ml d'ATG non hémadsorbée quand il faut 7,23 µg/ml d'ATG standard, soit 2,65 fois plus. L'étape d'hémadsorption diminue donc la capacité de liaison et l'activité spécifique des ATG.

### 3.2 Mesure de l'activité des ATG in vivo dans le test de greffe cutanée chez le singe

Trois groupes de singes ont été sélectionnés pour effectuer ce test : un groupe contrôle (n=5), un groupe recevant 5 mg/kg d'ATG standard (n=8) et un groupe recevant 5 mg/kg d'ATG non hémadsorbé (n=3) initialement. Une dilution de moitié des ATG non hémadsorbées est réalisée afin d'obtenir une activité spécifique équivalente à celle du lot standard.

Le test de greffe cutanée consiste à réaliser à J0 quatre allogreffes (provenant d'un animal dont au moins deux des déterminants antigéniques HLA sont différents) et une autogreffe de peau sur le dos de chaque animal. On administré ensuite *in vivo* les doses d'ATG pendant trois semaines à partir de J-1.

Les résultats montrent que les durées de survie moyenne des allogreffes sont équivalentes pour les deux groupes de singes traités : 23 jours (± 4,16) pour le groupe ATG standard et 21,3 jours (± 7,3) pour le groupe ATG non hémadsorbé. Elles sont en outre supérieures au contrôle : 9,4 jours (± 1,5).

L'allongement de la durée de survie de greffe cutanée est corrélé à la déplétion lymphocytaire induite par l'administration d'ATG. La lymphopénie suit une évolution comparable entre les deux lots : baisse maximale du taux de lymphocytes à J5 puis retour progressif aux valeurs normales vers J20.

En conséquence, le lot dilué d'ATG non hémadsorbées est équivalent en terme d'efficacité *in vivo* chez le primate au lot d'ATG standard non dilué.

### 3.3 Evaluation des risques anémiques hémolytiques consécutifs à l'administration d'ATG.

Dans le cadre de la même étude, des dosages sanguins ont été effectués régulièrement jusqu'à la fin du test et ont montré que l'évolution du taux d'hémoglobuline est identique chez les deux groupes de singes traités. On observe une diminution de ce taux jusqu'à J9, jusqu'à ce que celui-ci revienne progressivement aux valeurs normales. Deux phénomènes sont à l'origine de cette évolution : premièrement, l'anémie est de type iatrogène, consécutive aux prélèvements sanguins répétés. Cette anémie est identique dans les trois groupes de singes. Deuxièmement, un effet propre des ATG provoque une diminution sensible du taux d'hémoglobuline dès J1 dans les groupes traités, cette diminution étant identique avec l'ATG standard ou avec l'ATG non hémadsorbé.

De plus, afin de déceler une éventuelle origine hémolytique de l'anémie, les taux d'haptoglobine et d'oromucoïde sont également mesurés. En cas d'hémolyse, l'haptoglobine devient indosable. Or, ces deux taux augmentent dès J4 pour les deux groupes, augmentation qui est le reflet d'une activité inflammatoire.

Ainsi, la sécurité hématologique évaluée chez les animaux traités avec l'ATG non hémadsorbée est aussi satisfaisante que chez les animaux traités avec !'ATG hémadsorbée.

## Revendications

1. Procédé de production d'immunoglobulines anti-thymocytes humains qui comprend les étapes consistant à :
(a) injecter une préparation cellulaire de thymocytes humains à un animal non-humain exempt d'organismes pathogènes spécifiés (EOPS) ;
(b) recueillir le sérum immun produit par l'animal ;
(c) isoler les immunoglobulines anti-thymocytes humains du sérum, sans hémadsorption sur hématies, ni adsorption sur tissus humains, stroma ou extraits grossiers de ces tissus.

2. Procédé selon la revendication 1, dans lequel l'animal EOPS est un lapin.

3. Procédé selon l'une quelconque des revendications 1 ou 2, dans lequel l'étape d'isolement (c) met en oeuvre une chromatographie échangeuse d'ions, de préférence sur colonne, et/ou une ou plusieurs précipitations.

4. Procédé selon l'une quelconque des revendications 1 à 3, dans lequel la préparation cellulaire de thymocytes humains utilisée dans l'étape (a) est constituée de cellules de lignées en culture.

5. Procédé selon l'une quelconque des revendications 1 à 3, dans lequel la préparation cellulaire de thymocytes humains utilisée dans l'étape (a) est obtenue par purification de thymocytes humains frais.

## Patentansprüche

1. Verfahren zur Herstellung von Immunglobulinen, die gegen menschliche Thymozyten gerichtet sind, welches die folgenden Stufen umfaßt:
(a) Injizieren einer Zellpräparation von menschlichen Thymozyten einem nicht-menschlichen Tier, das frei von spezifischen pathogenen Spezies (EOPS) ist,
(b) Gewinnen des von dem Tier produzierten Immunserums,
(c) Isolieren der Immunglobuline, die gegen menschliche Thymozyten gerichtet sind, aus dem Serum ohne Hämadsorption auf roten Blutkörperchen noch auf menschlichem Gewebe, Stroma oder rohen Extrakten dieser Gewebe.

2. Verfahren gemäß Anspruch 1, bei welchem das von spezifischen pathogenen Spezies freie (EOPS) Tier ein Kaninchen ist.

3. Verfahren gemäß irgendeinem der Ansprüche 1 oder 2, bei welchem die Isolierstufe (c) eine Ionenaustauschchromatographie, bevorzugt in Kolonne, und/oder eine oder mehrere Ausfällungen anwendet.

4. Verfahren gemäß irgendeinem der Ansprüche 1 bis 3, bei welchem die in der Stufe (a) verwendete Zellpräparation von menschlichen Thymozyten durch Zelllinien in Kultur gebildet wird.

5. Verfahren gemäß irgendeinem der Ansprüche 1 bis 3, bei welchem die in der Stufe (a) verwendete Zellpräparation von menschlichen Thymozyten durch Reinigung von frischen menschlichen Thymozyten erhalten wurde.

## Claims

1. A method of producing human anti-thymocyte immunoglobulins, which comprises the steps consisting of :
(a) injecting a human thymocyte cell preparation to a non-human specific pathogen-free (SPF) animal ;
(b) collecting the immune serum produced by the animal ;
(c) isolating the human anti-thymocyte immunoglobulins from the serum, without hemadsorption on red blood cells, nor adsorption on human tissues, stroma or bulk extracts from these tissues.

2. Method according to claim 1, wherein the SPF animal is a rabbit.

3. Method according to any of claims 1 or 2, wherein the isolation step (c) is carried out with a ion exchange chromatography, preferably on a column, and/or one or several precipitations.

4. Method according to any of claims 1 to 3, wherein the human thymocyte cell preparation used in step (a) consists of cultured cell lines.

5. Method according to any of claims 1 to 3, wherein the human thymocyte cell preparation used in step (a) is obtained by purifying fresh human thymocytes.
